Europäisches Patentamt

European Patent Office

Office européen des brevets

(11) Numéro de publication: **0 242 371 B1**

# (12) FASCICULE DE BREVET EUROPEEN

(45) Date de publication de fascicule du brevet: **26.02.92**   (51) Int. Cl.⁵: **A61B 7/04**

(21) Numéro de dépôt: **86905706.7**

(22) Date de dépôt: **08.10.86**

(86) Numéro de dépôt internationale :
**PCT/CH86/00140**

(87) Numéro de publication internationale :
**WO 87/02233 (23.04.87 87/09)**

(54) STETHOSCOPE ACOUSTIOUE ET A FILTRE ELECTRIOUE.

(30) Priorité: **11.10.85 CH 4406/85**

(43) Date de publication de la demande:
**28.10.87 Bulletin 87/44**

(45) Mention de la délivrance du brevet:
**26.02.92 Bulletin 92/09**

(84) Etats contractants désignés:
**AT BE CH DE FR GB IT LI LU NL SE**

(56) Documents cités:
**DE-B- 1 084 428**
**FR-A- 2 363 317**
**US-A- 3 247 324**
**US-A- 3 651 798**

(73) Titulaire: **MAATEL S.A.**
**495, rue de Pommarin**
**F-38340 Voreppe(FR)**

(72) Inventeur: **Furugard, Eric**
**29, boulevard Helvétique**
**CH-1207 GENEVE(CH)**
Inventeur: **Caron, Charles**
**Le Châble Beaumont**
**F-74160 HAUTE-SAVOIE(FR)**

(74) Mandataire: **Ardin, Pierre et al**
**PIERRE ARDIN & CIE 22, rue du Mont-Blanc**
**CH-1201 Genève(CH)**

Rank Xerox (UK) Business Services

# Description

Les stéthoscopes sont beaucoup utilisés dans la médecine, mais ils demandent un certain entraînement. Ainsi, la mesure de la tension artérielle par "auscultation" peut être précise. Cependant, il faut noter l'influence non négligeable de l'opérateur sur les résultats de la mesure : acuité auditive, bruit environnant, temps de réaction, etc. Le point délicat est bien évidemment de déterminer la pression diastolique.

Dans certains appareils destinés à une mesure plus ou moins automatique de la tension artérielle, il est connu (voir US-A-3 651 798) de remplacer le stéthoscope habituel par une unité électrique comprenant un capteur, au moins un filtre d'entrée, un amplificateur et un dispositif à seuil de façon à fournir un signal électrique dès que le niveau des sons perçus par le capteur électrique, dans un domaine de fréquences donné, dépasse un seuil prédétermine. Il existe encore des stéthoscopes électriques basés sur le même principe, mais qui ne sont appréciés par le corps médical que dans certains cas particuliers. En effet, les médecins ont l'habitude du stéthoscope classique et ne veulent se fier uniquement à un stéthoscope électrique, ce qui les oblige à avoir souvent deux stéthoscopes sous la main, un de chaque espèce. Un stéthoscope pouvant fonctioner soit en mode acoustique soit en mode électronique est connu du FR-A-2 363 317.

La présente invention a pour but d'éviter les pertes de temps dues à l'emploi de deux stéthoscopes différents. Elle a pour objet un stéthoscope comprenant un capteur à membrane acoustique relié à des écouteurs par un tube acoustique, le capteur acoustique comprenant en outre un capteur électrique, relié par des conducteurs a une unité électrique comprenant au moins un filtre passe-bande d'entrée, un amplificateur, un dispositif à seuil et un émetteur électroacoustique destiné à transmettre un signal acoustique dès que le niveau des sons perçus par le capteur électrique, compris dans la plage des fréquences passantes du filtre dépasse un seuil prédéterminé, cet émetteur électroacoustique étant disposé pour transmettre son signal dans le tube acoustique, ce signal étant superposès aux sons acoustiques du stéthoscope.

Le dessin annexé représente schématiquement et à titre d'exemple une forme et des variantes d'exécution du stéthoscope faisant l'objet de l'invention.

La figure 1 est une vue extérieure d'un stéthoscope.

La figure 2 est un schéma électrique synoptique de l'unité électrique de ce stéthoscope.

Les figures 3 à 5 représentent différentes façons de relier l'émetteur électroacoustique au tube du stéthoscope.

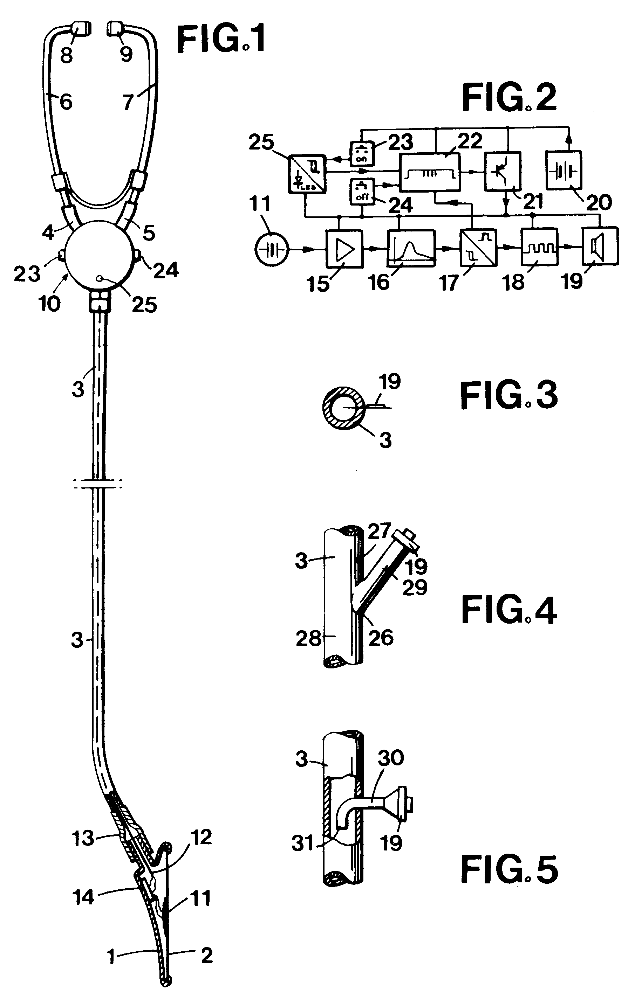

Le stéthoscope représenté à la figure 1 comprend, comme les stéthoscopes habituels, une capsule 1 fermée par une membrane souple 2 pour constituer un capteur acoustique. Cette capsule est reliée par un tube souple 3, se séparant en deux branches 4 et 5 aboutissant à deux tubes coudés 6 et 7, dont les extrémités supérieures sont munies d'embouts 8 et 9 destinés à être appliqués aux conduits auditifs de l'utilisateur.

Ce stéthoscope comprend en outre une unité électrique contenue dans un boîtier 10 qui est fixé sur le tube acoustique 3. Cette unité électrique est reliée à un capteur piezo-électrique 11 qui est fixé sur la membrane 2 du stéthoscope au moyen de colle. La liaison entre l'unité électrique et le capteur 11 est assurée par un câble blindé 12 passant dans le tube 3 et aboutissant à une plaquette de contact 14 fixée contre la paroi de la capsule 1 et reliée au capteur par deux conducteurs souples. Le tube 3 et le câble blindé 12 sont reliés au boîtier 10 par un connecteur 13.

La figure 2 montre le schéma de l'unité électrique qui est reliée au capteur 11. Le signal provenant du capteur est appliqué à un amplificateur 15, puis à un filtre 16 passe-bande qui atténue fortement les fréquences en dessous de 20 Hz et au dessus de 100 Hz. Les signaux qui traversent le filtre 16 sont ceux qui correspondent aux "bruits de Korotkoff" et parviennent à un Trigger de Schmitt 17 qui pilote un générateur 18 pour fournir des sons rythmés reproduits par un émetteur 19. L'alimentation de cet ensemble est assurée par une pile 20 dont le débit est contrôlé par un transistor 21. Ce dernier est commandé par une minuterie 22 électrique qui est commandée par un bouton poussoir 23 d'enclenchement et un bouton poussoir 24 de déclenchement pour rendre le transistor 21 conducteur, respectivement non conducteur, et alimenter ou non les circuits 15 à 19 et 24. Cette minuterie 22 est réactivée par le circuit 17 chaque fois que celui-ci émet une impulsion. L'état de la pile 20 peut être vérifié par un dispositif de contrôle 25 qui est rendu actif pendant la durée de fermeture du bouton poussoir 23.

L'émetteur 19 peut être associé de différentes façons au tube acoustique 3, pour que les signaux qu'il fournit soient entendus par l'utilisateur. Ainsi, à la figure 3, l'émetteur électroacoustique 19 est constitué par une plaquette vibrante collée et traversant la paroi du tube acoustique 3.

Dans le cas de la figure 4, le tube 3 présente un embranchement 26 en forme de Y dont une branche 27 est reliée au capteur acoustique, une branche 28 est reliée aux écouteurs et une branche 29 est reliée à l'émetteur 19. On voit que les branches 27 et 29 convergent vers la branche 28

qui est reliée aux écouteurs, de sorte que les sons provenant de l'émetteur **19** sont envoyés dans une direction générale correspondant au sens normal de la transmission des sons acoustiques dans le tube **3**.

La figure 5 montre une variante dans laquelle l'émetteur acoustique **19** est associé à un tuyau **30** traversant la paroi du tube acoustique **3** et coudé à l'intérieur de celui-ci pour que son extrémité **31** soit dirigée vers les écouteurs.

## Revendications

1. Stéthoscope comprenant un capteur à membrane (2) acoustique relié à des écouteurs par un tube acoustique (3), le capteur acoustique comprenant en outre un capteur électrique (11), relié par des conducteurs à unité électrique comprenant au moins un filtre (16) passe-bande d'entrée, un amplificateur (15), un dispositif à seuil (17) et un émetteur électroacoustique (19) destiné à transmettre un signal acoustique dès que le niveau des sons perçus par le capteur électrique, compris dans la plage des fréquences passantes du filtre dépasse un seuil prédéterminé, cet émetteur électroacoustique étant disposé pour transmettre son signal dans le tube acoustique (3), ce signal étant superposés aux sons acoustiques du stéthoscope.

2. Stéthoscope selon la revendication 1, **caractérisé** en ce que le tube acoustique présente un embranchement en forme de Y, avec une première branche reliée au capteur acoustique, une deuxième branche reliée aux écouteurs et une troisième branche reliée à l'émetteur électroacoustique.

3. Stéthoscope selon la renvendication 1 ou 2, **caractérisé** en ce que l'émmetteur électroacoustique est disposé contre la surface extérieure du tube de façon à transmettre son signal à travers la paroi du tube acoustique.

4. Stéthoscope selon la revendication 1 ou 2, **caractérisé** en ce que le tube acoustique présente un embranchement avec une première branche reliée au capteur acoustique, une deuxième branche reliée aux écouteurs et une troisième branche reliée à l'émetteur électroacoustique.

5. Stéthoscope selon la revendication 4, **caractérisé** en ce que l'embranchement est en forme de Y, dont la branche inférieure est reliée aux écouteurs.

6. Stéthoscope selon la revendication 1, **caractérisé** en ce que l'émetteur électroacoustique est associé à un tuyau traversant la paroi du tube acoustique et coudé à l'intérieur de celui-ci, pour que l'extrémité du tuyau soit dirigée vers les écouteurs.

## Claims

1. Stethoscope comprising an acoustic diaphragm sensor (2) connected to earpieces by an acoustic tube (3) the acoustic sensor comprising in addition an electrical sensor (11), connected by conductors to an electrical unit comprising at least an input band-pass filter (16), an amplifier (15), a threshold device (17) and an electroacoustic transmitter (14) designed to transmit an acoustic signal as soon as the level of sounds picked up by the electrical sensor, within the range of frequencies which the filter will pass, exceeds a predetermined threshold, this electroacoustic transmitter being arranged to transmit its signal in the acoustic tube (3), this signal being superimposed on the acoustic sounds of the stethoscope.

2. Stethoscope according to claim 1, characterised in that the acoustic tube has a Y-shaped junction, with a first branch connected to the acoustic sensor, a second branch connected to the earpieces and a third branch connected to the eloctroacoustic transmitter.

3. Stethoscope according to claim 1 or 2, characterised in that the electroacoustic transmitter is arranged against the external surface of the tube so as to transmit its signal through the wall of the acoustic tube.

4. Stethoscope according to claim 1 or 2, characterised in that the acoustic tube has a junction with a first branch connected to the acoustic sensor, a second branch connected to the earpieces and a third branch connected to the electroacoustic transmitter.

5. Stethoscope according to claim 4, characterised in that the junction is in the shape of a Y whose lower branch is connected to the earpieces.

6. Stethoscope according to claim 1, characterised in that the electroacoustic transmitter is associated with a pipe passing through the wall of the acoustic tube and bent inside the latter, so that the end of the pipe is directed towards the earpieces.

**Patentansprüche**

1.  Stethoskop mit einem eine akustische Membrane (2) aufweisenden Sensor, der mit Hörern durch einen Hörschlauch (3) verbunden ist und der außerdem einen elektrischen Meßfühler (11) enthält, der über Leiter mit einer elektrischen Einheit verbunden ist, die mindestens einen Eingangs-Bandpaßfilter (16), einen Verstärker (15), eine Schwellwerteinrichtung (17) und einen elektroakustischen Sender (19) zum Übertragen eines akustischen Signals aufweist, sobald das Niveau der durch den elektrischen Meßfühler empfangenen Töne des von dem Filter durchgelassenen Frequenzbandes eine vorbestimmte Schwelle überschreitet, wobei dieser elektroakustische Sender zum Übertragen seines Signals in den Hörschlauch (3) angeordnet ist und dieses Signal den akustischen Tönen des Stethoskops überlagert wird.

2.  Stethoskop nach Anspruch 1, **dadurch gekennzeichnet**, daß der Hörschlauch eine Y-förmige Verzweigung aufweist, mit einem ersten mit dem akustischen Sensor verbundenen Zweig, mit einem zweiten mit den Hörern verbundenen Zweig und mit einem dritten mit dem elektroakustischen Sender verbundenen Zweig.

3.  Stethoskop nach Anspruch 1 oder 2, **dadurch gekennzeichnet**, daß der elektroakustische Sender gegen die Außenfläche des Hörschlauchs zum Übertragen seines Signals durch die Wand des Hörschlauchs angeordnet ist.

4.  Stethoskop nach Anspruch 1 oder 2, **dadurch gekennzeichnet**, daß der Hörschlauch eine Verzweigung aufweist, mit einem ersten mit dem akustischen Sensor verbundenen Zweig, mit einem zweiten mit den Hörern verbundenen Zweig und mit einem dritten mit dem elektroakustischen Sender verbundenen Zweig.

5.  Stethoskop nach Anspruch 4, **dadurch gekennzeichnet**, daß die Verzweigung Y-förmig und ihr unterer Zweig mit den Hörern verbunden ist.

6.  Stethoskop nach Anspruch 1, **dadurch gekennzeichnet**, daß der elektroakustische Sender mit einem Schlauch vereinigt ist, der die Wand des Hörschlauchs durchdringt und in dessen Innerem so umgebogen ist, daß das Ende des Schlauchs in die Richtung der Hörer weist.

FIG.1

FIG.2

FIG.3

FIG.4

FIG.5